# EUROPEAN PATENT APPLICATION

(11) **EP 1 336 848 A1**
(43) Date of publication of application: **20.08.2003**
(21) Application number: 01934546.1
(22) Date of filing: 05.06.2001
(51) Int. Cl.: G01N 33/531

(54) **HIGHLY EFFICIENT METHOD OF SCREENING ANTIBODY**

(30) Priority: 24.11.2000 JP 2000358539
(71) Applicant: JAPAN SCIENCE AND TECHNOLOGY CORPORATION, Kawaguchi-shi, Saitama 332-0012 (JP); Japan as represented by President of National Center of Neurology and Psychiatry Ministry of Health, Kodaira-shi, Tokyo 187-8502 (JP)
(72) Inventor: KANEKO, Kiyotoshi, Kodaira-shi, Tokyo 187-0031 (JP)
(74) Representative: Harding, Charles Thomas
(86) International application number: JP0104732
(87) International publication number: WO02042774

(57) **Abstract**

The present invention provides a method in which antibodies against target proteins contained in samples are screened with high efficiency.

Samples including protein groups are treated with two-dimensional electrophoresis, the aforementioned protein groups are developed on a two-dimensional electrophoretic gel as an individual spot protein, the spot protein that had been developed is visualized after being transcribed onto a solid phase, then, after isolating each of the spot proteins, it is reacted with a phage antibody library, antibody that bonded to the spot proteins are replicated, an operation wherein said replicated antibody is reacted with the aforementioned spot protein is conducted once or more than twice, and antibodies against specific proteins contained in the samples including protein groups are screened. As samples including protein groups, it is preferable to use a samples wherein cellular subfraction had been separated and purified beforehand and samples wherein posttranslational modification had been removed.

## Description

### Technical Field

The present invention relates to a highly efficient antibody screening method, more specifically, a highly efficient antibody screening method wherein after protein mixtures of various cell fractions and the like are developed by two-dimensional electrophoresis, an antibody library is made to act on said mixtures, and a specific antibody is screened with high efficiency directly from the individual protein spot that had been separated.

### Background Art

With the rapid advance in the technique for base sequencing, the project to analyze the entire genome structure has come to the determination of the entire genome sequences in many microorganisms, and it is said that the project would be completed also in humans by the beginning of the next century. However, even if the base sequence is determined, there are only a few genes among the gene expression products, wherein its function can be predicted for example, by searching of homologous proteins with the use of databases. In addition, many of the proteins that actually express and function in the cell vary in many forms according to the condition of the cells, the change in its amount of expression and posttranslational modification. In this way, the set of proteins that exist in a certain instant in life is propounded as a new concept called proteome, which is expressed by a coined term of a combination of PROTEin and genOME (Kahn, P. Science 270, 369-70 (1995)). Analysis of the variation of proteome is conducted by comprehending the overall picture of proteome and comparing it with the overall picture of another certain instant. An attempt to analyze the biology by extensively analyzing the dynamics of these proteomes is called proteomics, and is drawing attention as a post genome project. Its method had already been established, and a method wherein two-dimensional electrophoresis and amino acid sequence analysis/mass spectrometry are combined is generally used.

Two-dimensional electrophoresis is known as a method wherein separation is conducted from two aspects of the characters that a substance has, for example, a method wherein electrophoresis according to isoelectric point is conducted beforehand, followed by electrophoresis according to molecular weight in different media (O'Farrell, P. H., J. Biol. Chem. 250. 4007-21 (1975)). According to said two-dimensional electrophoresis, since it is developed two-dimensionally based on the two characters, isoelectric point and molecular weight, it is developed in a different coordinate if the isoelectric point differs, even if the substances have the same molecular weight, thereby enabling each substance to separate as a spot on the two-dimensional coordinate. Two-dimensional electrophoresis is frequently used especially as a method for separating proteins, because of its excellence in resolution. The two-dimensional electrophoresis method of proteins that is currently used is conducted generally in the following manner. First, isoelectric point electrophoresis is conducted by using separation media such as capillary gel and commercially available strip gel, the gel which electrophoresis had been completed is mounted on a second planate SDS-polyacrylamide gel (slab gel), and electrophoresis is conducted to the direction perpendicular to the developing direction of isoelectric point electrophoresis. As just described, many proteomes have already been analyzed by two-dimensional electrophoresis, wherein an isoelectric point electrophoresis and a SDS-polyacrylamide electrophoresis (SDS-PAGE) are combined, that is, separation to a one-dimensional direction according to the isoelectric point, and then development to a two-dimensional direction according to molecular weight are conducted consecutively. Regarding each protein spot that had been separated from the protein group, there have already been many reports regarding the analysis results from amino acid sequence determination and peptide mass fingerprinting, together with the coordinate information.

On the other hand, to obtain an antibody against a desired protein holds an important position for identification and functional analysis of the protein. However, the conventional method in which a desired antigen is administered to animals for immunization requires various and large quantities of antigens, a long time, and a large amount of effort. Thus, in recent years, a method has been established to construct an antibody by using a phage antibody library. Said method is an application of a system wherein an antibody is presented (displayed) on the surface of a phage by fusion of antibody with a coat protein of a filamentous phage, and a phage display library can be made by constructing a library including a variety of antibody genes by amplification of antibody gene with PCR, and then presenting this on the phage. According to this method, it is also possible to construct an antibody against antigens of human. The screening method wherein said phage antibody library is used is called a phage display method, and has been used conventionally to identify ligands and various antibodies that bind specifically to various cell surface receptors. There have already been many reports also regarding the construction method of a phage antibody library and screening methods using said method such as immunotube method, magnetic beads method, and the like (Scott, J. M. and Smith, G. P., Science, 249, 386-390 (1990); Smith, G. P. and Scott, J. K., Methods in Enzymology, 217, 228-257 (1993)).

As previously described, regarding proteomics which has drawn attention as a post genome project, a technique wherein two-dimensional electrophoresis and amino acid sequence analysis/mass spectrometry are combined is in heavy usage, however, it has been pointed out that there are several problems in said technique. In two-dimensional electrophoresis, there are problems such as the limit to the amount of sample attachments, the complexity of analysis due to the enormous numbers of spots, the limit to the separation power at the acidic and basic sides, reproducibility, and the limit to the spot detection sensitivity. Although quantitative minimization of substances to be analyzed has progressed in amino acid sequence analysis/mass spectrometry, it still remains unsatisfactory, and is said that skill is required for mass spectrometry analysis. An object of the present invention is to provide a highly efficient method of screening antibody against target proteins contained in the sample.

The inventors of the present invention had conducted intensive studies to attain the object mentioned above, and focused attention on the point that a library having a diversity of 10⁹ has already been constructed as a phage antibody library, and that it can theoretically cover all kinds of proteins. It has been discovered that 2D phage panning method (2D-PP), which is a combination of phage panning method that uses said phage antibody library and two-dimensional electrophoresis, can be a new proteome analysis method that can sufficiently oppose to the combination of two-dimensional electrophoresis and mass spectrometry that is currently the main method for proteome analysis. More specifically, protein spot that had been separated by two-dimensional electrophoresis was transcribed to a nitrocellulose membrane, only the spot portion was cut out after visualization by gold colloid staining, only said spot portion was reacted with a phage antibody library, only the phage antibody that bound specifically to the desired protein spot was retrieved, said collected phage antibody was infected to Escherichia coli, said specific phage antibody was produced in large quantities, and the reaction and retrieval operations mentioned above were further repeated, in other words, it was discovered that monoclonal antibody against a desired protein spot can be screened with high efficiency by panning, and thus the present invention had been completed.

### Disclosure of the Invention

More specifically, the present invention relates to a highly efficient antibody screening method, wherein the method is a screening method of antibody against a specific protein contained in the sample including a protein group, comprising the following processes (a) to (c), (a) a process wherein a sample including a protein group is treated with two-dimensional electrophoresis, and the aforementioned protein group is developed as an individual spot protein on the two-dimensional electrophoretic gel, (b) a process wherein the spot proteins and antibody libraries are reacted, and (c) a process wherein the antibodies bonded to the spot proteins are replicated, and the replicated antibodies are reacted with the aforementioned spot proteins once or more than twice (claim 1); the highly efficient antibody screening method according to claim 1, wherein samples where its cellular subfractions had been separated and purified beforehand are used as samples including protein groups (claim 2); the highly efficient antibody screening method according to claim 1 or 2, wherein samples in which posttranslational modification had been eliminated are used as the samples including protein groups (claim 3); the highly efficient antibody screening method according to any of claims 1 to 3, wherein the individual spot proteins that had been developed on the two-dimensional electrophoretic gel are reacted with the antibody libraries as spot proteins that have been transcribed onto a solid phase (claim 4); the highly efficient antibody screening method according to claim 4, wherein the spot proteins that had been transcribed onto the solid phase are visualized, and after isolating each of the spot proteins, they are reacted with the antibody libraries (claim 5); the highly efficient antibody screening method according to any of claims 1 to 5, wherein a phagemid antibody library is used as the antibody library (claim 6); the highly efficient antibody screening method according to claim 6, wherein the phagemid antibodies bonded to the spot proteins are infected with host cells, and phagemid antibodies are replicated (claim 7); and the highly efficient antibody screening method according to claim 6, wherein PCR is conducted by targeting the CDR region of the phagemid antibodies bonded to the spot proteins, followed by infecting a phagemid vector incorporating the region amplified by PCR with host cells, and replicating phagemid antibodies (claim 8).

### Brief Description of Drawing

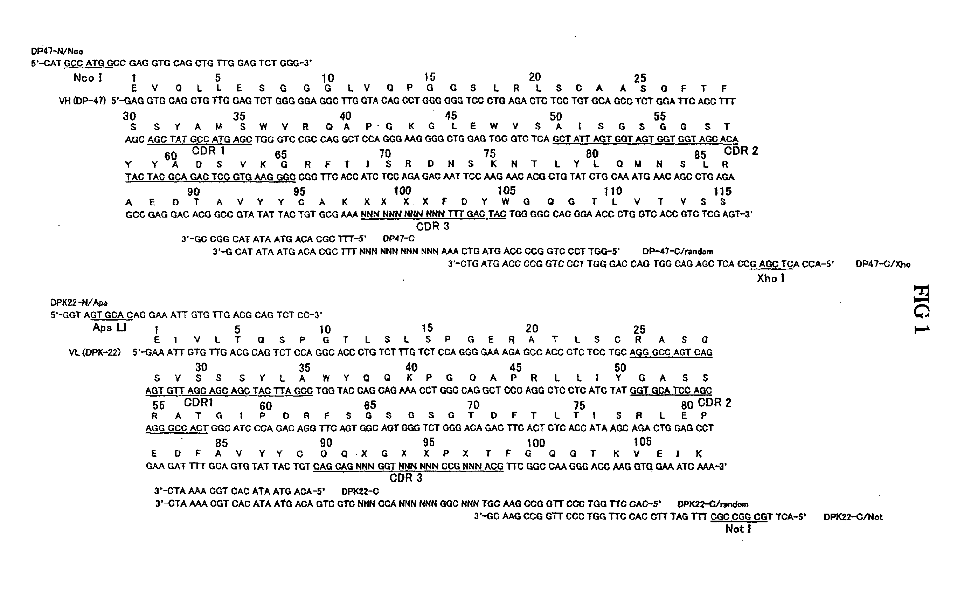

Figure 1 is a view indicating the sequence information of DP-47 and DPK-22.

### Best Mode of Carrying Out the Invention

In the highly efficient antibody screening method of the present invention, there is no particular limitation as long as it is a screening method of antibodies against specific proteins contained in the samples including protein groups, comprising (a) a process wherein a sample including a protein group is treated with two-dimensional electrophoresis, and the aforementioned protein group is developed as an individual spot protein on the two-dimensional electrophoretic gel, (b) a process wherein the spot proteins and antibody libraries are reacted, and (c) a process wherein the antibodies bonded to the spot proteins are replicated, and the replicated antibodies are reacted with the aforementioned spot proteins once or more than twice. Moreover, the above-mentioned antibodies include immunoglobulin, single strand antibody, and antibody fragments including antibody-binding sites comprised of variable region of antibodies. Exemplification of antibody fragments are F(ab')₂ obtained by peptonizing monoclonal antibodies, Fab' obtained by reduction of F(ab')₂, Fab obtained by digesting monoclonal antibodies with papain, and the like.

As a sample including the protein groups mentioned above, it can be anything as long as it is a sample including several kinds of proteins, however, it is preferable for the sample to be able to develop as individual spot proteins on the two-dimensional electrophoretic gel, when said sample is treated with two-dimensional electrophoresis. Treatment with two-dimensional electrophoresis in the usual manner enables to separate the sample into several thousand protein spots at a maximum, however, the separation power by said treatment with two-dimensional electrophoresis cannot cover all the proteins that are said to be tens of thousands and that are expressed and functioned in the cells. As just described, if samples treated with two-dimensional electrophoresis exceed the detection limit as a protein spot, it is preferable to conduct separation and purification, fractionation treatment and the like beforehand, to make it possible to be detected as a protein spot. For example, it is preferable to separate and purify the targeted cellular subfraction (caveolae, clathrin-coated pits, Golgi, lysosome and the like) beforehand by biochemical methods or the like, and develop said protein groups that had been separated and purified as starting materials, as individual spot proteins on the two-dimensional electrophoresis. By said pre-treatment, it is possible to develop the several hundreds to several thousands of protein groups that exist in the individual cellular subfraction as individual spot proteins, even with the separation power of a normal two-dimensional electrophoresis treatment. Exemplification of samples including protein groups wherein said cellular subfractions had been separated and purified beforehand are, for example, fractions comprised of cell membrane microdomain "lipid rafts" that are structured by various kinds of membrane proteins abundant in intercellular and intracellular signaling system molecules and indicate high hydrophobic properties that are obtained by separating and purifying human neuroblastoma, or a clathrin-coated pits fraction that comprise many receptor proteins, or cell membrane fractions that do not belong to them but are involved in important intercellular signaling, and the like.

Moreover, as samples including protein groups mentioned above, it is preferable to use samples wherein the posttranslational modification of proteins (for example, glycosylation, phosphorylation, acetylation, amidation, myristoylation, farnesylation, geranylgeranylation, sulfation, and glycophosphatidyl inositol anchor (GPI anchor)) had been eliminated. By the treatment of eliminating the posttranslational modification of said proteins, it is also made possible to separate proteins that do not become spots under a condition of a regular two-dimensional electrophoresis. In addition, there is no particular limitation to the method for eliminating the posttranslational modification, but it can be conducted by accordingly combining enzyme treatment such as protein phosphatase (phosphoproteinphosphatase), methylesterase, peptide N-glycosidase F (PNGaseF), phosphatidylinositol phospholipase C (PIPLC) or the like, and degreasing treatment by chemical compounds or the like.

As a two-dimensional electrophoresis treatment wherein protein groups contained in the samples are developed as individual spot proteins on the two-dimensional electrophoretic gel, there is no particular limitation as long as it is a method that conducts separation from the two aspects of physical property, such as isoelectric point and molecular weight, that the proteins have. Generally, it can be done by first conducting isoelectric point electrophoresis with the use of separation media such as capillary gel or commercially available strip gel, followed by mounting the gel wherein electrophoresis had been completed on a second planate SDS-polyacrylamide gel (slab gel), and conducting electrophoresis to the direction perpendicular to the developing direction of isoelectric point electrophoresis. It is preferable to transcribe the individual spot proteins that had developed on the two-dimensional electrophoretic gel by means of said treatment of two-dimensional electrophoresis, onto a solid phase such as nitrocellulose membrane, polyvinylidene difluoride membrane (PVDF membrane) or the like, by methods such as wet method of Western Blotting or the like, and react the spot proteins transcribed onto said solid phase and the antibody libraries. By the transcription onto said solid phase, the target protein spots and antibody libraries can easily be reacted.

It is much more preferable to visualize the spot proteins transcribed onto the solid phase mentioned above, isolate each of the spot proteins, and then react them with the antibody libraries. In each of the spots isolated after said visualization, antibodies other than the antibodies against the desired proteins, such as antibodies against solid phase membrane (nitrocellulose membrane, PVDF membrane or the like) can be excluded, by elimination of the parts where a protein does not exist, and antibodies against target proteins can be screened with high efficiency. There is no particular limitation to the method for visualizing the spot proteins mentioned above, as long as it is a method for visualizing proteins that are publicly known, such as gold colloid staining method, immunostaining method and the like. Moreover, there is also no particular limitation to the method for isolating each of the spot proteins by cutting out after visualization. However, in order to increase the reproducibility, it is preferable to use a method in which only the spot portions are cut out, by using an automatic cutting apparatus that is commercially available, such as a spot cutter or the like.

As to an antibody library used in the present invention, there is no particular limitation as long as it is a library including a number of antibodies, but it is preferable to use a phagemid antibody library (phage display library) comprised of about 109 phagemid antibodies with wide range of variation, that can theoretically correspond to the antigenic epitopes on every protein. As said phagemid antibody library, any kind of library can be used as long as it is a phage library that is publicly known used in a normal phage display method, including those that are commercially available. For example, a phage display human antibody library can be constructed in the following manner: a gene fragment that encodes the variable regions (V_{H}, V_{L}) of H and L chain of the antibody is-amplified by PCR from human B cells; said amplified genes introduce the filamentous phage (phagemid vector) that had been inserted within the framework of its coat protein pIII genes to the Escherichia coli, and helper phages such as VCS-13 and the like are infected with said Escherichia coli (H. R. Hoogenboom et al., Immunotechnology, 4, 1-20 (1998)).

Moreover, as a phage antibody library, a library named Sequence-arrayed library (SAL), which had been constructed by the present inventors, can be used advantageously. The description of SAL will be followed hereinafter. It is known that there is a Fab structure that is used preferentially in a phage antibody that specifically recognizes antigens in a phage antibody library, wherein VH chain is DP47 and VL chain is DPK22. Furthermore, in the CDR region of these chains, it is also known that a certain limited site (VH chain: amino acids in 95th, 96th, 97th, and 98th residues/ VL chain: amino acids in 91st, 93rd, 94th, and 96th residues) of CDR3 region in particular, is deeply involved with the binding to the antigens. Consequently, the frequency of usage of amino acids used in these regions was examined, and it was discovered that there is a difference in the frequency of usage. In addition, these amino acids could be limited to seven types of amino acids (G, S, D, N, R, Y, P) when classified according to their properties. It was discovered that placing these amino acids in a random order in the antigen binding sites of CDR3 region enables to construct a library having a variety of 5.8x10⁶, and that the amount of one type of phage antibody can be increased from the conventional 10⁴ to 10⁶.

Consequently, a primer design method which covers its diversity and being highly effective was examined, and it was discovered that the seven types of amino acids mentioned above can be substituted by three types of codons. When the three types of codons are placed at an antigen-binding site, they can cover all the diversities with 81 types of primers for DP47 (Seq. ID Nos. 1 to 81) and 81 types of primers for DPK22 (Seq. ID Nos. 82 to 162). Furthermore, in these primers, it can be made possible also to increase the amount of one type of phage antibody to more than 10⁶, by reducing the numbers of amino acids used and by constructing a sub-library by combining DP47 and DPK22 that had been synthesized with each of the primers. Accordingly, this SAL is capable not only of screening the phage antibody against very minute amounts of antigens but also of screening phage antibody where panning is not necessary. In addition, with the use of this SAL, if the antigen can be cloned even when the specific property is low, it is possible to strengthen the specific properties by affinity maturation.

The reaction of the aforementioned spot protein and a phagemid antibody library can be conducted by ordinary method, for example, by using approximately 5x10¹² cfu/mL phage antibody. After reacting a spot protein with a phagemid antibody library, when only the phagemid antibody attached to the desired individual spot proteins is retrieved and infected with Escherichia coli, phagemid antibodies that react with the individual spots can be replicated in a large amount. Since the DNA sequence information of the antibody portion of phagemid antibody is incorporated into the phagemid, when the phagemid is replicated by infection of Escherichia coli, antibodies that are expressed on its surface are also replicated at the same time, the antibodies that react with the desired individual protein spots can be replicated at a large amount and easily. In fact, it is rare that a positive antibody can be identified with reaction conducted once, and antibodies against the desired individual spot proteins can be obtained by repeating this operation several times. This cycle of repetition is called "panning". Usually, phage antibodies that bind specifically to the target proteins are concentrated by repeating said panning several times, preferably by repetition of three to five times, and screening can be conducted.

As another method for the above-mentioned panning, in place of a method wherein phagemid is infected directly with the Escherichia coli, a method can be given wherein PCR is conducted by targeting the CDR region (mainly CDR3) of the phagemid antibody bonded to the individual spot proteins, a phagemid vector wherein the region amplified by PCR has been incorporated is infected with host cells, and a phagemid antibody is replicated. The advantage of this method is that since the size of the targeted CDR region is nearly constant and the primer sequence used for PCR is common, the uneven of amplification by PCR, which is generally a big problem when conducting replication by PCR, can be suppressed to the minimum. By actually using this method, it can achieve approximately ten times the efficiency compared to the method wherein phagemid is infected directly with Escherichia coli.

In addition, the following can be given as methods for eliminating antibodies against unfavorable solid phase membrane or the like, when identifying positive antibodies, by the reaction of a spot protein with a phagemid antibody library, aside from the previously mentioned method wherein only the individual spot portion is isolated: a method wherein different types of solid phase membranes (nitrocellulose membrane and PVDF membrane or the like) are used alternatively every time panning is conducted (Alternative membrane method), a method wherein a phagemid antibody library is reacted with the solid phase membrane or the like beforehand; and the antibodies against these are eliminated, and a method wherein these methods are combined, and the like.

It is preferable to perform immunoblotting on the two-dimensional electrophoresis by using positive antibodies against each target protein obtained by the above-mentioned screening method, and confirm the reactivity and specificity between the desired individual spot proteins and each antibody. Moreover, it is possible to make the antibody monoclonal by examining the correlation between the DNA footprint and the antibody titer of each antibody.

In addition, it is possible to prepare homogeneous phagemid antibodies at a large amount very easily, by infecting the positive antibodies obtained by a screening with Escherichia coli. Furthermore, when infected with specific Escherichia coli strain such as the Escherichia coli HB2151 strain or the like, these phagemid antibodies are released from the Escherichia coli and can be solubilized in the solution as an antibody fragment. When preparing suchlike solubilized antibody fragment, it is advantageous to prepare it as a fusion peptide having a sequence tag such as c-Myc or His-tag, in consideration of the purification of the solubilized antibody fragment. In addition, by fixing the substance that specifically recognizes this tag on the chip beforehand, and then reacting each solubilized antibody fragment that had been solubilized with the chip, these antibody fragments can easily be fixed on the chip. An antibody chip can be made by fractionating the surface of the chip in pieces and fixing different phagemid antibodies to each of the fractions. Moreover, after samples had been solubilized under various conditions, the samples wherein protein had been labeled by the use of fluorescent probe and chemiluminescence probe or the like, are reacted with the above-mentioned antibody chip under the same condition as the usual established antigen antibody reaction, followed by the measurement of the amount of labeled probes that are remained, thereby the amount of expression of the proteins contained in the samples can be determined. It is made possible to analyze together the expression profile of proteins contained in the object samples, by measuring together the amount of remaining labeled probes that correspond to each fraction on the antibody chip.

By a screening with the use of the positive antibodies thus identified and the expression library of proteins, it is made possible to detect positive clone on the expression library that reacts with positive antibodies. Since the base sequence and the translated amino acid sequence of said positive clone are homologous with the protein sequence of each spot that is developed on the two-dimensional electrophoresis, the amino acid sequence information of the targeted individual proteins and the amino acid sequence information can be obtained. When this screening is conducted, the tertiary structure of both proteins of samples and an expression library can be made analogous by denaturing the protein of the expression library under the same condition as two-dimensional electrophoresis, thereby enabling to limit the problem of tertiary structure in an antigen antibody reaction to the minimum. In addition, since the above-mentioned protein expression library lacks posttranslational modification from the beginning, it is much preferable to use proteins wherein the posttranslational modification of the proteins contained in the sample is removed as a starting material.

The present invention will now be explained more specifically with the following examples, however, the technical scope of the invention is not limited to these examples.

### Example 1 [Preparation of target protein fraction]

As a target protein fraction, a cell membrane microdomain "lipid rafts" that are comprised of various kinds of membrane proteins abundant in intercellular and intracellular signaling system molecules and indicate high hydrophobic properties, were fractionated from human neuroblastoma and used. A human neuroblast SK-N-MC (ATCC No. HTB10) that had been incubated for 5 days was retrieved from an Eagle's MEM medium wherein 10% of fetal bovine serum and L-glutamine and penicillin/streptomycin had been added. After suspending in a lysis buffer (25 mM MES; pH6.5, 150mMNaCl, 1% (v/v) TritonX-100, protease inhibitors), cells were fractured by reciprocating the pestle 15 times by using a homogenizer (Iuchi), to prepare a cell lysate. An equivalent volume of 80% sucrose/MES-buffered saline (25 mM MES; pH 6.5, 150 mM NaCl) was added to the cell lysate, followed by injection to the bottom of the centrifuge tube after adjusting the concentration of the sucrose to 40%, and after superposition of sucrose gradient from 5% to 30%, it was set in a horizontal type roter (Beckman Sw41Ti). A sucrose density gradient centrifugation was conducted by using an ultracentrifuge (Beckman Optima L-70K Ultracentrifuge) at 35000 rpm for 24 hours at 4 . degree. C. A target protein fraction (protein concentration approximately 120 µg/ml) was prepared by retrieving fraction abundant in lipid rafts that were converged in the vicinity of 15% sucrose concentration.

### Example 2 [Two-dimensional electrophoresis]

The target protein fraction obtained from Example 1 was separated and developed by two-dimensional electrophoresis comprised of isoelectric point electrophoresis and SDS-PAGE. The isoelectric point electrophoresis was conducted by using an Immobiline Dry Strip (Amersham Pharmacia) as an isoelectric point gel, and an IPGphor (Amersham Pharmacia) was used as an electrophoretic instrument. The target protein fraction was lysed to an electrophoretic solution comprised of 9M Urea, 2M Thiourea, 4% CHAPS, 20 mM Tris-HCL, and 0.5% IPG buffer, and after re-swelling for 6 hours, voltage was then applied in the order of 30 V for 6 hours, 500 V for 1 hour, 1000 V for 1 hour, and 8000 V for 12 hours. The isoelectric point gel was then shaken in a SDS equilibrating solution for 20 minutes, and adhered onto the SDS-PAGE gel, and subsequently, SDS-PAGE was conducted. SDS-PAGE was conducted by Hoefer SE600 (Amersham Pharmacia) and was electrified at 20 mA for 5 hours. From the operations mentioned above, the protein group included in the target protein fraction was separated and developed to be the individual protein spots.

### Example 3 [Solid phasing of target protein spot]

The individual protein spots that were separated and developed in Example 2 were transcribed and solid phased on a nitrocellulose membrane (Amersham Pharmacia, Hybond-ECL) by a common wet-type Western Blotting. Western Blotting was performed by using TE62X (Amersham Pharmacia), by electrification at 150 mA for 16 hours. The nitrocellulose membrane for Western Blotting was shaken for 1 hour in a gold colloid stain solution (Bio-Rad), and the protein spot that had been solid phased was visualized. 50 spots that had been visualized were cut off by a Spot Cutter (Bio-Rad), and were made to be an antigen sample for phage display method.

### Example 4 [Selection of phage antibody that recognizes target proteins]

The solid phased material of the target protein that had been prepared in Example 3 was transferred to a 2 mL-capacity Eppendorf (Ep) tube, and a PBS containing 1% Tween 20 was first added, and was shaken gently for 1 hour at room temperature (renature). The solution was then exchanged to PBS including 10% skim milk and 0.1% Tween 20 (MPBST), and was shaken gently for 1 hour at room temperature (blocking). After this solution was excluded, 5% MPBST (total of 800 µL) including 300 µL each of media supernatant of Escherichia coli HB2151 strain that has secreted two types of solubilized anti-nitrocellulose antibody fragment, that was prepared by the same method as shown in Example 7 that will be described hereinafter, was added, and was shaken gently for 2 hours at room temperature. After this had been excluded, 200 µL of 5% MPBST containing a phage antibody with a concentration wherein the absorbance at wavelength 260 nm is 5 (which is equivalent to approximately 5x10¹² cfu/mL) (a total of 4 to 5 rounds of panning was implemented, wherein a library phage was used for panning at first round, and the phage retrieved from the previous round was used for the second round and after) and the above-mentioned solubilized anti-nitrocellulose antibody fragment (25µL each of two types of media supernatant) was added, and was shaken and reacted for 3 hours at room temperature. A human synthetic Fv library "Griffin. 1" (provided by Dr. G. Winter of MRC Great Britain), which is a phagemid-based antibody library having a diversity of more than 10⁹, was used for the phage library at the first round of panning. This reactant solution was shaken 3 times for 10 minutes by PBS including 0.1% Tween 20, washed once for 10 minutes in the same manner by PBS, and eliminated the non-specific reactive phage.

The phage that bonded specifically to the target protein was eluted by shaking with 100 µL of 100 mM triethylamine for 10 minutes, and this solution was transferred to 1.5 mL-capacity Ep tube containing 50 µL of 1M-Tris·HCl (pH 8.0) and then neutralized. In addition, 20 µL of 1 M-Tris·HCl (pH 8.0) was added to a tube containing spots after the phage had been eluted, and the spots were also neutralized. On the contrary, Escherichia coli TG1 strain being cultured until the absorbance at wavelength 600 nm changed from 0.5 to 1.0, was added 850 µL to the phage eluted solution tube and 400 µL to the tube containing spots, respectively, and the tubes were left at rest for 30 minutes at 37.degree. C. to infect the phage. A total of 1420 µL which is a mixture of these, was plated to LB plate containing 100 µg/mL ampicillin and 1% glucose (including 10 g trypton, 5 g yeast extract, 10 g NaCl, 15g agar in 1 L) (LB/Amp Glc·plate), and was cultured overnight at 30.degree. C. to form a colony.

### Example 5 [Preparation of phage antibody]

The entire colony of Escherichia coli TG1 strain on the LB/Amp·Glc plate according to Example 4 was scraped, and suspended in 25 mL of 2xTY containing 100 µg/mL ampicillin and 1% glucose (containing 16 g trypton, 10 g yeast extract, and 5 g NaCl in 1 L) (2xTY/Amp·Glc), such that the absorbance at wavelength 600 nm was arranged to be lower than approximately 1.0, and was spinner cultured at 37.degree. C. for 1 hour. 3 mL of the mixture was transferred to a tube containing 2.4×10¹⁰ pfu helper phage VCS-M13 (Stratagene) and was left at rest at 37.degree. C. for 30 minutes, and the supernatant was removed by centrifuging for 10 minutes at 3250×g. The Escherichia coli that had precipitated was suspended in 12.5 mL of 2×TY containing 100 µg/mL ampicillin and 25 µg/mL kanamycin (2×TY/Amp·Kan), and was spinner cultured overnight at 30.degree. C. After eliminating the precipitation by centrifuging this cultured solution for 10 minutes at 10750xg at 4.degree. C., PEG/NaCl solution (containing 20% polyethylene glycol and 2.5 M NaCl), one-fifth of the culture supernatant in volume, was added to the cultured supernatant and mixed, and was left for more than 30 minutes at 4.degree. C. After conducting centrifugation at 10750xg for 10 minutes at 4.degree. C and the resultant was further centrifuged at 10750xg and the supernatant was completely aspirated and was removed. The precipitated phage was dissolved in 0.5 mL PBS, and this was centrifuged for 10 minutes at 11600xg to precipitate the insoluble matter, and the supernatant was transferred to another tube to obtain a phage antibody solution. The phage antibody solution thus obtained had a concentration providing approximately 10 to 15 of absorbance at wavelength 260 nm.

### Example 6 [Verification of specificity by Western Blotting]

A monoclonal phage antibody can also be prepared from the polyclonal phage antibody according to Example 5. A single colony on a LB/Amp·Glc plate after panning was inoculated into 100 µL of 2xTY/Amp·Glc in a 96 well microtiter plate made of polypropylene, and shaking culture was conducted overnight at 37.degree. C. Secondly, 2 µL of the culture was inoculated into 200 µL of 2xTY/Amp·Glc in a 96 well microtiter plate made of polypropylene, followed by 2 hours of shaking culture at 37.degree. C., and was then added 50 µL 2xTYIAmp·Glc containing 2x10⁹ pfu of VCS-M13, and was then left at rest for 30 minutes at 37.degree. C. This was centrifuged at 2380xg at room temperature for 15 minutes to remove the supernatant, the bacterial was suspended in 1.8 mL of 2xTY/Amp in a Falcon 14 mL round tube, and was cultured overnight at 30.degree. C. 1.5 mL of the supernatant was used in the same manner as in Example 5 to obtain a phage antibody solution (100 to 200 µL).

The monoclonal phage antibody thus obtained or the polyclonal phage antibody according to Example 5 was used to verify its specificity by Western Blotting. That is, "lipid rafts" which had been developed to one-dimensional (SDS-PAGE) or two-dimensional (isoelectric point electrophoresis and SDS-PAGE), and had been transcribed into nitrocellulose membrane (Amersham Pharmacia, Hybond-ECL), was first renatured for 1 hour with PBS containing 10% of Tween 20, and was further blocked for 1 hour with 10% MPBST. After it was washed with deionized water, 10% of MPBST containing a phage antibody with a concentration such that the absorbance at wavelength 260 nm is made to be 0.05 was added, and the resultant solution was shaken and reacted at room temperature for 1 hour. This membrane was washed with deionized water for 3 times, and further washed for 5 minutes each for 3 times with PBST. Then, HRP (horseradish peroxidase)-labeled anti-M13 antibody (Amersham Pharmacia) diluted to 7000 times was added to 5% MPBST, and was shaken at room temperature for 30 minutes to 1 hour. This was washed 3 times for 5 minutes each with PBST, washed once for 5 minutes with PBST, and after ECL detecting reagent was added, a film was exposed to its chemiluminescent signal. It was verified herewith that the polyclonal and monoclonal phage antibodies selected through the panning operation reacts with high specificity to the target protein spots used in screening. Moreover, since this sequential operation showed the same effectiveness also in other multiple spots as the result of verification, it was then verified that specific antibodies can be obtained directly through screening a phage antibody library from the protein groups that have been two-dimensionally separated and solid phased on the membrane.

### Example 7 (Preparation of solubilized antibody fragment]

The phage antibody clone whose specificity had been verified in Example 6 was added to a culture solution of Escherichia coli HB2151 strain cultured such that the absorbance at wavelength 600 nm is changed from 0.5 to 1.0 in 1 mL of 2xTY/Glc (1%), and the resultant mixture was left at rest for 30 minutes at 37.degree. C. Ampicillin was added to the mixture such that a final concentration was arranged to be 100 µg/mL and shaking culture was conducted overnight at 37.degree. C. 0.5 mL of this mixture was inoculated into 50 mL of 2xTY/Amp·Glc (0.1%) which had been kept warm at 37.degree. C. beforehand, shaking culture was conducted at 37.degree. C. until the end of logarithmic growth phase, and IPTG (isopropyl β -D thiogalactopyranoside) was added to the mixture such that a final concentration was arranged to be 1 mM to induce expression, shaking culture was conducted overnight at 30.degree. C., and solubilized antibody fragment was secreted in the medium. This was centrifuged for 10 minutes at 10750xg to eliminate the bacterial, and a medium supernatant containing solubilized antibody fragment was obtained. Since this solubilized antibody fragment has a tag comprised of 6 residues of histidine in its carboxyl-terminals, this molecule was purified according to its need by metal chelate affinity chromatography using this tag. That is, after passing the medium supernatant that contained solubilized antibody fragment in a neutral condition to a column filled with TALON resin (CLONTECH), it was washed with Wash buffer (containing 50 mM sodium phosphate pH 7.0, 300 mM NaCl, and 5 mM imidazole), and the antibody fragment that had been absorbed was eluted with Elution buffer (containing 50 mM sodium phosphate pH 7.0, 300 mM NaCl, and 5 mM imidazole), and a purified reference preparation was obtained by eluting the antibody fragment with Elution buffer (containing 50 mM sodium phosphate pH 7.0, 300 mM NaCl, and 150 mM imidazole).

### Example 8 [Cloning of VH chain DP47 and VL chain DPK22]

In order to construct a SAL phage antibody library, cloning of VH chain DP47 clone and VL chain DPK22 clone (DP47-DPK22 Sequence: Seq. ID No. 163), which are starting materials, was conducted based on the aforementioned human synthetic Fv library "Griffin.1 (see Figure 1). PCR was conducted by using a phagemid vector and a primer [DP47-N/Nco (Seq. ID No. 165) and DP47-C (Seq. ID No. 166) for DP47 (Seq. ID No.164), and DPK22-N/Apa (Seq. ID No. 168) and DPK22-C (Seq. ID No. 169) for DPK22 (Seq. ID No.167)], VH chain DP47 and VL chain DPK22 were amplified, and after cloning with the use of TA-cloning vector, the phagemid was retrieved and sequencing was conducted (DP47-4 (Seq. ID No. 170) and DPK22). Since the expected sequence could not be obtained for VH chain DP47 clone, a primer for introducing a new mutation [DP47-5'-1 (Seq. ID No. 171), -3'-1 (Seq. ID No. 172), -5'-2 (Seq. ID No. 173), -3'-2 (Seq. ID No. 174)] was synthesized, and a point mutation was introduced by PCR method with the use of QuikChange Site-Directed Mutagenesis Kit (Stratagene), sequencing was conducted again, and mutation was verified (Mutated DP47). Herewith, although the base sequence of Mutated DP47 (Seq. ID No. 175) was different from DP47, a clone homologous to the expected amino acid sequence was obtained. Based on these clones, amino acid mutation was introduced in the manner described in Example 9 to the amino acids in eight important portions (four parts each in VH chain Mutated DP47 and VL chain DPK 22) in anitigen antibody reaction, and a SAL phage antibody library was constructed.

### Example 9 [Construction of phage antibody library SAL]

81 types of primers comprised of base sequence indicated as Seq. ID Nos. 1 to 81 as primers for VH chain DP47, and 81 types of primers comprised of base sequence indicated as Seq. ID Nos. 82 to 162 as primers for VL chain DPK22 were synthesized in the usual manner. PCR was conducted by using these primers, followed by (1) construction of random region (in eight important portions in the antigen antibody reaction) [DP47-N/Nco and DP47-C/random (Seq. ID Nos. 1 to 81), and DPK22-N/Apa and DPK22-C/random (Seq. ID Nos. 82 to 162)], and (2) addition of restriction enzyme site necessary to incorporation into the phagemid [DP47-N/Nco and DP47-C/Xho (Seq. ID No. 176), and DPK22-N/Apa and DPK22-C/Not (Seq. ID No. 177)]. By these primers, one among the seven types of amino acids (G, S, D, N, R, Y, P) is arranged randomly in the 95th, 96th, 97th, and 98th residues of VH chain DP47 indicated as "NNN" in Figure 1, and the 91st, 93rd, 94th, and 96th residues of VL chain DPK22. The VH chain DP47 and VL chain DPK were synthesized by PCR method with the use of these primers, and after these amplified fragments were cut by restriction enzyme, a phage display library was constructed according to the usual manner. Herewith, a sequence-arrayed library wherein a single chain antibody fragment with a diversity of 10⁶ is presented on the surface of the filamentous phage was obtained. In addition, the "NNN" in DP47-C/random primer and DPK22-C/random primer shown in Figure 1 are selected from one among ATH (H is C, T or A; ATC=Asp, ATT=Asn, ATA=Tyr) , ACB (B is T, G or C; ACT=Ser, ACG=Arg, ACC=Gly), and CGG (Pro). However, since the library constructed according to the above-mentioned Example used the combination only of ATH and ACB, each primer would use 3x3x3x3=81 types of synthesized primer. In addition, since there are 16 combinations of ATH and ACB for each of DP47 and DPK22, it makes the diversity of this library a total of (81×16)×(81×16)=1.7×l06.

### Industrial Applicability

When analyzing individual proteins for their localization and detailed functions, and interaction that affects other molecules and cells, their specific antibodies are indespensable. According to the present invention, antibodies against target proteins contained in the samples can be screened with high efficiency, and by using the antibodies obtained, in addition to the identification of target proteins, studies of activity inhibition of desired protein molecules and introduction of stimulus into cells by using the specific antobodies obtained can be conducted. This is very effective when promoting future research on proteomics. In addition, a cDNA expression library of proteins can be screened by using the antibodies that had been obtained from screening. Proteins can effectively be identified from protein groups that were separated by two-dimensional electrophoresis, without the use of the conventional proteomics analysis method such as mass spectrometry, amino acid sequence, or the like. Moreover, according to the present invention, since the target proteins do not go into the condition wherein the whole molecules are exposed in the aqueous phase, through the entire reaction system in antibody screening, it can ignore the hydrophilicity of antigens generally required in the phage antibody system, and further screen the cDNA expression library of proteins by using antibodies obtained by screening, thereby enabling to establish the full length sequence, even when the target spot protein is a part of the fractionated protein.

## Claims

1. A highly efficient antibody screening method, wherein the method is a screening method of antibody against a specific protein contained in the sample including a protein group, comprising the following processes (a) to (c),
(a) a process wherein a sample including a protein group is treated with two-dimensional electrophoresis, and the aforementioned protein group is developed as an individual spot protein on the two-dimensional electrophoretic gel,
(b) a process wherein the spot proteins and antibody libraries are reacted, and
(c) a process wherein the antibodies bonded to the spot proteins are replicated, and the replicated antibodies are reacted with the aforementioned spot proteins once or more than twice.

2. The highly efficient antibody screening method according to claim 1, wherein samples where its cellular subfractions had been separated and purified beforehand are used as samples including protein groups.

3. The highly efficient antibody screening method according to claim 1 or 2, wherein samples in which posttranslational modification had been eliminated are used as the samples including protein groups.

4. The highly efficient antibody screening method according to any of claims 1 to 3, wherein the individual spot proteins that had been developed on the two-dimensional electrophoretic gel are reacted with the antibody libraries as spot proteins that have been transcribed onto a solid phase.

5. The highly efficient antibody screening method according to claim 4, wherein the spot proteins that had been transcribed onto the solid phase are visualized, and after isolating each of the spot proteins, they are reacted with the antibody libraries.

6. The highly efficient antibody screening method according to any of claims 1 to 5, wherein a phagemid antibody library is used as the antibody library.

7. The highly efficient antibody screening method according to claim 6, wherein the phagemid antibodies bonded to the spot proteins are infected with host cells, and phagemid antibodies are replicated.

8. The highly efficient antibody screening method according to claim 6, wherein PCR is conducted by targeting the CDR region of the phagemid antibodies bonded to the spot proteins, followed by infecting a phagemid vector incorporating the region amplified by PCR with host cells, and replicating phagemid antibodies.


SEQUENCE LISTING

<110> JAPAN SCIENCE AND TECHNOLOGY CORPORATION
Japan as Represented by President of National Center of Neurology
and Psychiatry
Ministry of Health, Labour and Welfare

<120> High efficiency screening method for antibodies

<130> A081-04PCT

<140>
<141>

<150> 2000-358539
<151> 2000-11-24

<160> 177

<170> PatentIn Ver. 2.1

<210> 1
<211> 55
<212> DNA
<213> Artificial Sequence

<220>
<223> Description of Artificial Sequence:DP47-C/random

<400> 1
ggttccctgg ccccagtagt caaaathath athathtttc gcacagtaat atacg 55

<210> 2
<211> 55
<212> DNA
<213> Artificial Sequence

<220>
<223> Description of Artificial Sequence:DP47-C/random

<400> 2
ggttccctgg ccccagtagt caaaathath athacbtttc gcacagtaat atacg 55

<210> 3

<211> 55
<212> DNA
<213> Artificial Sequence

<220>
<223> Description of Artificial Sequence:DP47-C/random

<400> 3
ggttccctgg ccccagtagt caaaathath athcggtttc gcacagtaat atacg     55


<210> 4
<211> 55
<212> DNA
<213> Artificial Sequence

<220>
<223> Description of Artificial Sequence:DP47-C/random

<400> 4
ggttccctgg ccccagtagt caaaathath acbathtttc gcacagtaat atacg     55


<210> 5
<211> 55
<212> DNA
<213> Artificial Sequence

<220>
<223> Description of Artificial Sequence:DP47-C/random

<400> 5
ggttccctgg ccccagtagt caaaathath acbacbtttc gcacagtaat atacg     55


<210> 6
<211> 55
<212> DNA
<213> Artificial Sequence

<220>
<223> Description of Artificial Sequence:DP47-C/random

<400> 6
ggttccctgg ccccagtagt caaaathath acbcggtttc gcacagtaat atacg     55

<210> 7
<211> 55
<212> DNA
<213> Artificial Sequence

<220>
<223> Description of Artificial Sequence:DP47-C/random

<400> 7
ggttccctgg ccccagtagt caaaathath cggathtttc gcacagtaat atacg      55


<210> 8
<211> 55
<212> DNA
<213> Artificial Sequence

<220>
<223> Description of Artificial Sequence:DP47-C/random

<400> 8
ggttccctgg ccccagtagt caaaathath cggacbtttc gcacagtaat atacg      55


<210> 9
<211> 55
<212> DNA
<213> Artificial Sequence

<220>
<223> Description of Artificial Sequence:DP47-C/random

<400> 9
ggttccctgg ccccagtagt caaaathath cggcggtttc gcacagtaat atacg      55


<210> 10
<211> 55
<212> DNA
<213> Artificial Sequence

<220>
<223> Description of Artificial Sequence:DP47-C/random

<400> 10

ggttccctgg ccccagtagt caaaathacb athathtttc gcacagtaat atacg     55

<210> 11
<211> 55
<212> DNA
<213> Artificial Sequence

<220>
<223> Description of Artificial Sequence:DP47-C/random

<400> 11
ggttccctgg ccccagtagt caaaathacb athacbtttc gcacagtaat atacg     55

<210> 12
<211> 55
<212> DNA
<213> Artificial Sequence

<220>
<223> Description of Artificial Sequence:DP47-C/random

<400> 12
ggttccctgg ccccagtagt caaaathacb athcggtttc gcacagtaat atacg     55

<210> 13
<211> 55
<212> DNA
<213> Artificial Sequence

<220>
<223> Description of Artificial Sequence:DP47-C/random

<400> 13
ggttccctgg ccccagtagt caaaathacb acbathtttc gcacagtaat atacg     55

<210> 14
<211> 55
<212> DNA
<213> Artificial Sequence

<220>
<223> Description of Artificial Sequence:DP47-C/random

<400> 14
ggttccctgg ccccagtagt caaaathacb acbacbtttc gcacagtaat atacg      55


<210> 15
<211> 55
<212> DNA
<213> Artificial Sequence

<220>
<223> Description of Artificial Sequence:DP47-C/random

<400> 15
ggttccctgg ccccagtagt caaaathacb acbcggtttc gcacagtaat atacg      55


<210> 16
<211> 55
<212> DNA
<213> Artificial Sequence

<220>
<223> Description of Artificial Sequence:DP47-C/random

<400> 16
ggttccctgg ccccagtagt caaaathacb cggathtttc gcacagtaat atacg      55


<210> 17
<211> 55
<212> DNA
<213> Artificial Sequence

<220>
<223> Description of Artificial Sequence:DP47-C/random

<400> 17
ggttccctgg ccccagtagt caaaathacb cggacbtttc gcacagtaat atacg      55


<210> 18
<211> 55
<212> DNA
<213> Artificial Sequence

<220>
<223> Description of Artificial Sequence:DP47-C/random

<400> 18
ggttccctgg ccccagtagt caaaathacb cggcggtttc gcacagtaat atacg      55


<210> 19
<211> 55
<212> DNA
<213> Artificial Sequence

<220>
<223> Description of Artificial Sequence:DP47-C/random

<400> 19
ggttccctgg ccccagtagt caaaathcgg athathtttc gcacagtaat atacg      55


<210> 20
<211> 55
<212> DNA
<213> Artificial Sequence

<220>
<223> Description of Artificial Sequence:DP47-C/random

<400> 20
ggttccctgg ccccagtagt caaaathcgg athacbtttc gcacagtaat atacg      55


<210> 21
<211> 55
<212> DNA
<213> Artificial Sequence

<220>
<223> Description of Artificial Sequence:DP47-C/random

<400> 21
ggttccctgg ccccagtagt caaaathcgg athcggtttc gcacagtaat atacg      55


<210> 22
<211> 55
<212> DNA

<213> Artificial Sequence

<220>
<223> Description of Artificial Sequence:DP47-C/random

<400> 22
ggttccctgg ccccagtagt caaaathcgg acbathtttc gcacagtaat atacg     55


<210> 23
<211> 55
<212> DNA
<213> Artificial Sequence

<220>
<223> Description of Artificial Sequence:DP47-C/random

<400> 23
ggttccctgg ccccagtagt caaaathcgg acbacbtttc gcacagtaat atacg     55


<210> 24
<211> 55
<212> DNA
<213> Artificial Sequence

<220>
<223> Description of Artificial Sequence:DP47-C/random

<400> 24
ggttccctgg ccccagtagt caaaathcgg acbcggtttc gcacagtaat atacg     55


<210> 25
<211> 55
<212> DNA
<213> Artificial Sequence

<220>
<223> Description of Artificial Sequence:DP47-C/random

<400> 25
ggttccctgg ccccagtagt caaaathcgg cggathtttc gcacagtaat atacg     55


<210> 26

<211> 55
<212> DNA
<213> Artificial Sequence

<220>
<223> Description of Artificial Sequence:DP47-C/random

<400> 26
ggttccctgg ccccagtagt caaaathcgg cggacbtttc gcacagtaat atacg      55


<210> 27
<211> 55
<212> DNA
<213> Artificial Sequence

<220>
<223> Description of Artificial Sequence:DP47-C/random

<400> 27
ggttccctgg ccccagtagt caaaathcgg cggcggtttc gcacagtaat atacg      55


<210> 28
<211> 55
<212> DNA
<213> Artificial Sequence

<220>
<223> Description of Artificial Sequence:DP47-C/random

<400> 28
ggttccctgg ccccagtagt caaaacbath athathtttc gcacagtaat atacg      55


<210> 29
<211> 55
<212> DNA
<213> Artificial Sequence

<220>
<223> Description of Artificial Sequence:DP47-C/random

<400> 29
ggttccctgg ccccagtagt caaaacbath athacbtttc gcacagtaat atacg      55

<210> 30
<211> 55
<212> DNA
<213> Artificial Sequence

<220>
<223> Description of Artificial Sequence:DP47-C/random

<400> 30
ggttccctgg ccccagtagt caaaacbath athcggtttc gcacagtaat atacg      55


<210> 31
<211> 55
<212> DNA
<213> Artificial Sequence

<220>
<223> Description of Artificial Sequence:DP47-C/random

<400> 31
ggttccctgg ccccagtagt caaaacbath acbathtttc gcacagtaat atacg      55


<210> 32
<211> 55
<212> DNA
<213> Artificial Sequence

<220>
<223> Description of Artificial Sequence:DP47-C/random

<400> 32
ggttccctgg ccccagtagt caaaacbath acbacbtttc gcacagtaat atacg      55


<210> 33
<211> 55
<212> DNA
<213> Artificial Sequence

<220>
<223> Description of Artificial Sequence:DP47-C/random

<400> 33

ggttccctgg ccccagtagt caaaacbath acbcggtttc gcacagtaat atacg      55

<210> 34
<211> 55
<212> DNA
<213> Artificial Sequence

<220>
<223> Description of Artificial Sequence:DP47-C/random

<400> 34
ggttccctgg ccccagtagt caaaacbath cggathtttc gcacagtaat atacg      55

<210> 35
<211> 55
<212> DNA
<213> Artificial Sequence

<220>
<223> Description of Artificial Sequence:DP47-C/random

<400> 35
ggttccctgg ccccagtagt caaaacbath cggacbtttc gcacagtaat atacg      55

<210> 36
<211> 55
<212> DNA
<213> Artificial Sequence

<220>
<223> Description of Artificial Sequence:DP47-C/random

<400> 36
ggttccctgg ccccagtagt caaaacbath cggcggtttc gcacagtaat atacg      55

<210> 37
<211> 55
<212> DNA
<213> Artificial Sequence

<220>
<223> Description of Artificial Sequence:DP47-C/random

<400> 37
ggttccctgg ccccagtagt caaaacbacb athathtttc gcacagtaat atacg     55


<210> 38
<211> 55
<212> DNA
<213> Artificial Sequence

<220>
<223> Description of Artificial Sequence:DP47-C/random

<400> 38
ggttccctgg ccccagtagt caaaacbacb athacbtttc gcacagtaat atacg     55


<210> 39
<211> 55
<212> DNA
<213> Artificial Sequence

<220>
<223> Description of Artificial Sequence:DP47-C/random

<400> 39
ggttccctgg ccccagtagt caaaacbacb athcggtttc gcacagtaat atacg     55


<210> 40
<211> 55
<212> DNA
<213> Artificial Sequence

<220>
<223> Description of Artificial Sequence:DP47-C/random

<400> 40
ggttccctgg ccccagtagt caaaacbacb acbathtttc gcacagtaat atacg     55


<210> 41
<211> 55
<212> DNA
<213> Artificial Sequence

<220>
<223> Description of Artificial Sequence:DP47-C/random

<400> 41
ggttccctgg ccccagtagt caaaacbacb acbacbtttc gcacagtaat atacg 55


<210> 42
<211> 55
<212> DNA
<213> Artificial Sequence

<220>
<223> Description of Artificial Sequence:DP47-C/random

<400> 42
ggttccctgg ccccagtagt caaaacbacb acbcggtttc gcacagtaat atacg 55


<210> 43
<211> 55
<212> DNA
<213> Artificial Sequence

<220>
<223> Description of Artificial Sequence:DP47-C/random

<400> 43
ggttccctgg ccccagtagt caaaacbacb cggathtttc gcacagtaat atacg 55


<210> 44
<211> 55
<212> DNA
<213> Artificial Sequence

<220>
<223> Description of Artificial Sequence:DP47-C/random

<400> 44
ggttccctgg ccccagtagt caaaacbacb cggacbtttc gcacagtaat atacg 55


<210> 45
<211> 55
<212> DNA

<213> Artificial Sequence

<220>
<223> Description of Artificial Sequence:DP47-C/random

<400> 45
ggttccctgg ccccagtagt caaaacbacb cggcggtttc gcacagtaat atacg      55


<210> 46
<211> 55
<212> DNA
<213> Artificial Sequence

<220>
<223> Description of Artificial Sequence:DP47-C/random

<400> 46
ggttccctgg ccccagtagt caaaacbcgg athathtttc gcacagtaat atacg      55


<210> 47
<211> 55
<212> DNA
<213> Artificial Sequence

<220>
<223> Description of Artificial Sequence:DP47-C/random

<400> 47
ggttccctgg ccccagtagt caaaacbcgg athacbtttc gcacagtaat atacg      55


<210> 48
<211> 55
<212> DNA
<213> Artificial Sequence

<220>
<223> Description of Artificial Sequence:DP47-C/random

<400> 48
ggttccctgg ccccagtagt caaaacbcgg athcggtttc gcacagtaat atacg      55


<210> 49

<211> 55
<212> DNA
<213> Artificial Sequence

<220>
<223> Description of Artificial Sequence:DP47-C/random

<400> 49
ggttccctgg ccccagtagt caaaacbcgg acbathtttc gcacagtaat atacg 55


<210> 50
<211> 55
<212> DNA
<213> Artificial Sequence

<220>
<223> Description of Artificial Sequence:DP47-C/random

<400> 50
ggttccctgg ccccagtagt caaaacbcgg acbacbtttc gcacagtaat atacg 55


<210> 51
<211> 55
<212> DNA
<213> Artificial Sequence

<220>
<223> Description of Artificial Sequence:DP47-C/random

<400> 51
ggttccctgg ccccagtagt caaaacbcgg acbcggtttc gcacagtaat atacg 55


<210> 52
<211> 55
<212> DNA
<213> Artificial Sequence

<220>
<223> Description of Artificial Sequence:DP47-C/random

<400> 52
ggttccctgg ccccagtagt caaaacbcgg cggathtttc gcacagtaat atacg 55

<210> 53
<211> 55
<212> DNA
<213> Artificial Sequence

<220>
<223> Description of Artificial Sequence:DP47-C/random

<400> 53
ggttccctgg ccccagtagt caaaacbcgg cggacbtttc gcacagtaat atacg      55


<210> 54
<211> 55
<212> DNA
<213> Artificial Sequence

<220>
<223> Description of Artificial Sequence:DP47-C/random

<400> 54
ggttccctgg ccccagtagt caaaacbcgg cggcggtttc gcacagtaat atacg      55


<210> 55
<211> 55
<212> DNA
<213> Artificial Sequence

<220>
<223> Description of Artificial Sequence:DP47-C/random

<400> 55
ggttccctgg ccccagtagt caaacggath athathtttc gcacagtaat atacg      55


<210> 56
<211> 55
<212> DNA
<213> Artificial Sequence

<220>
<223> Description of Artificial Sequence:DP47-C/random

<400> 56

ggttccctgg ccccagtagt caaacggath athacbtttc gcacagtaat atacg 55

<210> 57
<211> 55
<212> DNA
<213> Artificial Sequence

<220>
<223> Description of Artificial Sequence:DP47-C/random

<400> 57
ggttccctgg ccccagtagt caaacggath athcggtttc gcacagtaat atacg 55

<210> 58
<211> 55
<212> DNA
<213> Artificial Sequence

<220>
<223> Description of Artificial Sequence:DP47-C/random

<400> 58
ggttccctgg ccccagtagt caaacggath acbathtttc gcacagtaat atacg 55

<210> 59
<211> 55
<212> DNA
<213> Artificial Sequence

<220>
<223> Description of Artificial Sequence:DP47-C/random

<400> 59
ggttccctgg ccccagtagt caaacggath acbacbtttc gcacagtaat atacg 55

<210> 60
<211> 55
<212> DNA
<213> Artificial Sequence

<220>
<223> Description of Artificial Sequence:DP47-C/random

<400> 60
ggttccctgg ccccagtagt caaacggath acbcggtttc gcacagtaat atacg      55


<210> 61
<211> 55
<212> DNA
<213> Artificial Sequence

<220>
<223> Description of Artificial Sequence:DP47-C/random

<400> 61
ggttccctgg ccccagtagt caaacggath cggathtttc gcacagtaat atacg      55


<210> 62
<211> 55
<212> DNA
<213> Artificial Sequence

<220>
<223> Description of Artificial Sequence:DP47-C/random

<400> 62
ggttccctgg ccccagtagt caaacggath cggacbtttc gcacagtaat atacg      55


<210> 63
<211> 55
<212> DNA
<213> Artificial Sequence

<220>
<223> Description of Artificial Sequence:DP47-C/random

<400> 63
ggttccctgg ccccagtagt caaacggath cggcggtttc gcacagtaat atacg      55


<210> 64
<211> 55
<212> DNA
<213> Artificial Sequence

<220>
<223> Description of Artificial Sequence:DP47-C/random

<400> 64
ggttccctgg ccccagtagt caaacggacb athathtttc gcacagtaat atacg     55


<210> 65
<211> 55
<212> DNA
<213> Artificial Sequence

<220>
<223> Description of Artificial Sequence:DP47-C/random

<400> 65
ggttccctgg ccccagtagt caaacggacb athacbtttc gcacagtaat atacg     55


<210> 66
<211> 55
<212> DNA
<213> Artificial Sequence

<220>
<223> Description of Artificial Sequence:DP47-C/random

<400> 66
ggttccctgg ccccagtagt caaacggacb athcggtttc gcacagtaat atacg     55


<210> 67
<211> 55
<212> DNA
<213> Artificial Sequence

<220>
<223> Description of Artificial Sequence:DP47-C/random

<400> 67
ggttccctgg ccccagtagt caaacggacb acbathtttc gcacagtaat atacg     55


<210> 68
<211> 55
<212> DNA

<213> Artificial Sequence

<220>
<223> Description of Artificial Sequence:DP47-C/random

<400> 68
ggttccctgg ccccagtagt caaacggacb acbacbtttc gcacagtaat atacg     55



<210> 69
<211> 55
<212> DNA
<213> Artificial Sequence

<220>
<223> Description of Artificial Sequence:DP47-C/random

<400> 69
ggttccctgg ccccagtagt caaacggacb acbcggtttc gcacagtaat atacg     55



<210> 70
<211> 55
<212> DNA
<213> Artificial Sequence

<220>
<223> Description of Artificial Sequence:DP47-C/random

<400> 70
ggttccctgg ccccagtagt caaacggacb cggathtttc gcacagtaat atacg     55



<210> 71
<211> 55
<212> DNA
<213> Artificial Sequence

<220>
<223> Description of Artificial Sequence:DP47-C/random

<400> 71
ggttccctgg ccccagtagt caaacggacb cggacbtttc gcacagtaat atacg     55



<210> 72

<211> 55
<212> DNA
<213> Artificial Sequence

<220>
<223> Description of Artificial Sequence:DP47-C/random

<400> 72
ggttccctgg ccccagtagt caaacggacb cggcggtttc gcacagtaat atacg      55


<210> 73
<211> 55
<212> DNA
<213> Artificial Sequence

<220>
<223> Description of Artificial Sequence:DP47-C/random

<400> 73
ggttccctgg ccccagtagt caaacggcgg athathtttc gcacagtaat atacg      55


<210> 74
<211> 55
<212> DNA
<213> Artificial Sequence

<220>
<223> Description of Artificial Sequence:DP47-C/random

<400> 74
ggttccctgg ccccagtagt caaacggcgg athacbtttc gcacagtaat atacg      55


<210> 75
<211> 55
<212> DNA
<213> Artificial Sequence

<220>
<223> Description of Artificial Sequence:DP47-C/random

<400> 75
ggttccctgg ccccagtagt caaacggcgg athcggtttc gcacagtaat atacg      55

<210> 76
<211> 55
<212> DNA
<213> Artificial Sequence

<220>
<223> Description of Artificial Sequence:DP47-C/random

<400> 76
ggttccctgg ccccagtagt caaacggcgg acbathtttc gcacagtaat atacg      55


<210> 77
<211> 55
<212> DNA
<213> Artificial Sequence

<220>
<223> Description of Artificial Sequence:DP47-C/random

<400> 77
ggttccctgg ccccagtagt caaacggcgg acbacbtttc gcacagtaat atacg      55


<210> 78
<211> 55
<212> DNA
<213> Artificial Sequence

<220>
<223> Description of Artificial Sequence:DP47-C/random

<400> 78
ggttccctgg ccccagtagt caaacggcgg acbcggtttc gcacagtaat atacg      55


<210> 79
<211> 55
<212> DNA
<213> Artificial Sequence

<220>
<223> Description of Artificial Sequence:DP47-C/random

<400> 79

ggttccctgg ccccagtagt caaacggcgg cggathtttc gcacagtaat atacg      55


<210> 80
<211> 55
<212> DNA
<213> Artificial Sequence

<220>
<223> Description of Artificial Sequence:DP47-C/random

<400> 80
ggttccctgg ccccagtagt caaacggcgg cggacbtttc gcacagtaat atacg      55


<210> 81
<211> 55
<212> DNA
<213> Artificial Sequence

<220>
<223> Description of Artificial Sequence:DP47-C/random

<400> 81
ggttccctgg ccccagtagt caaacggcgg cggcggtttc gcacagtaat atacg      55


<210> 82
<211> 63
<212> DNA
<213> Artificial Sequence

<220>
<223> Description of Artificial Sequence:DPK22-C/random

<400> 82
caccttggtc ccttggccga acgtathcgg athathacca thctgctgac agtaatacac 60
tgc                                                               63


<210> 83
<211> 63
<212> DNA
<213> Artificial Sequence

<220>

<223> Description of Artificial Sequence:DPK22-C/random

<400> 83
caccttggtc ccttggccga acgtathcgg athathacca cbctgctgac agtaatacac 60
tgc 63


<210> 84
<211> 63
<212> DNA
<213> Artificial Sequence

<220>
<223> Description of Artificial Sequence:DPK22-C/random

<400> 84
caccttggtc ccttggccga acgtathcgg athathaccc ggctgctgac agtaatacac 60
tgc 63


<210> 85
<211> 63
<212> DNA
<213> Artificial Sequence

<220>
<223> Description of Artificial Sequence:DPK22-C/random

<400> 85
caccttggtc ccttggccga acgtathcgg athacbacca thctgctgac agtaatacac 60
tgc 63


<210> 86
<211> 63
<212> DNA
<213> Artificial Sequence

<220>
<223> Description of Artificial Sequence:DPK22-C/random

<400> 86
caccttggtc ccttggccga acgtathcgg athacbacca cbctgctgac agtaatacac 60
tgc 63

<210> 87
<211> 63
<212> DNA
<213> Artificial Sequence

<220>
<223> Description of Artificial Sequence:DPK22-C/random

<400> 87
caccttggtc ccttggccga acgtathcgg athacbaccc ggctgctgac agtaatacac 60
tgc 63


<210> 88
<211> 63
<212> DNA
<213> Artificial Sequence

<220>
<223> Description of Artificial Sequence:DPK22-C/random

<400> 88
caccttggtc ccttggccga acgtathcgg athcggacca thctgctgac agtaatacac 60
tgc 63


<210> 89
<211> 63
<212> DNA
<213> Artificial Sequence

<220>
<223> Description of Artificial Sequence:DPK22-C/random

<400> 89
caccttggtc ccttggccga acgtathcgg athcggacca cbctgctgac agtaatacac 60
tgc 63


<210> 90
<211> 63
<212> DNA
<213> Artificial Sequence

<220>
<223> Description of Artificial Sequence:DPK22-C/random

<400> 90
caccttggtc ccttggccga acgtathcgg athcggaccc ggctgctgac agtaatacac 60
tgc 63

<210> 91
<211> 63
<212> DNA
<213> Artificial Sequence

<220>
<223> Description of Artificial Sequence:DPK22-C/random

<400> 91
caccttggtc ccttggccga acgtathcgg acbathacca thctgctgac agtaatacac 60
tgc 63

<210> 92
<211> 63
<212> DNA
<213> Artificial Sequence

<220>
<223> Description of Artificial Sequence:DPK22-C/random

<400> 92
caccttggtc ccttggccga acgtathcgg acbathacca cbctgctgac agtaatacac 60
tgc 63

<210> 93
<211> 63
<212> DNA
<213> Artificial Sequence

<220>
<223> Description of Artificial Sequence:DPK22-C/random

<400> 93
caccttggtc ccttggccga acgtathcgg acbathaccc ggctgctgac agtaatacac 60
tgc 63

<210> 94

<211> 63
<212> DNA
<213> Artificial Sequence

<220>
<223> Description of Artificial Sequence:DPK22-C/random

<400> 94
caccttggtc ccttggccga acgtathcgg acbacbacca thctgctgac agtaatacac 60
tgc 63


<210> 95
<211> 63
<212> DNA
<213> Artificial Sequence

<220>
<223> Description of Artificial Sequence:DPK22-C/random

<400> 95
caccttggtc ccttggccga acgtathcgg acbacbacca cbctgctgac agtaatacac 60
tgc 63


<210> 96
<211> 63
<212> DNA
<213> Artificial Sequence

<220>
<223> Description of Artificial Sequence:DPK22-C/random

<400> 96
caccttggtc ccttggccga acgtathcgg acbacbaccc ggctgctgac agtaatacac 60
tgc 63


<210> 97
<211> 63
<212> DNA
<213> Artificial Sequence

<220>
<223> Description of Artificial Sequence:DPK22-C/random

<400> 97
caccttggtc ccttggccga acgtathcgg acbcggacca thctgctgac agtaatacac 60
tgc                                                                63

<210> 98
<211> 63
<212> DNA
<213> Artificial Sequence

<220>
<223> Description of Artificial Sequence:DPK22-C/random

<400> 98
caccttggtc ccttggccga acgtathcgg acbcggacca cbctgctgac agtaatacac 60
tgc                                                                63

<210> 99
<211> 63
<212> DNA
<213> Artificial Sequence

<220>
<223> Description of Artificial Sequence:DPK22-C/random

<400> 99
caccttggtc ccttggccga acgtathcgg acbcggaccc ggctgctgac agtaatacac 60
tgc                                                                63

<210> 100
<211> 63
<212> DNA
<213> Artificial Sequence

<220>
<223> Description of Artificial Sequence:DPK22-C/random

<400> 100
caccttggtc ccttggccga acgtathcgg cggathacca thctgctgac agtaatacac 60
tgc                                                                63

<210> 101
<211> 63

<212> DNA
<213> Artificial Sequence

<220>
<223> Description of Artificial Sequence:DPK22-C/random

<400> 101
caccttggtc ccttggccga acgtathcgg cggathacca cbctgctgac agtaatacac 60
tgc 63


<210> 102
<211> 63
<212> DNA
<213> Artificial Sequence

<220>
<223> Description of Artificial Sequence:DPK22-C/random

<400> 102
caccttggtc ccttggccga acgtathcgg cggathaccc ggctgctgac agtaatacac 60
tgc 63


<210> 103
<211> 63
<212> DNA
<213> Artificial Sequence

<220>
<223> Description of Artificial Sequence:DPK22-C/random

<400> 103
caccttggtc ccttggccga acgtathcgg cggacbacca thctgctgac agtaatacac 60
tgc 63


<210> 104
<211> 63
<212> DNA
<213> Artificial Sequence

<220>
<223> Description of Artificial Sequence:DPK22-C/random

<400> 104
   cacctiggtc ccttggccga acgtathcgg cggacbacca cbctgctgac agtaatacac 60
   tgc                                                                63

<210> 105
<211> 63
<212> DNA
<213> Artificial Sequence

<220>
<223> Description of Artificial Sequence:DPK22-C/random

<400> 105
caccttggtc ccttggccga acgtathcgg cggacbaccc ggctgctgac agtaatacac 60
tgc                                                                63

<210> 106
<211> 63
<212> DNA
<213> Artificial Sequence

<220>
<223> Description of Artificial Sequence:DPK22-C/random

<400> 106
caccttggtc ccttggccga acgtathcgg cggcggacca thctgctgac agtaatacac 60
tgc                                                                63

<210> 107
<211> 63
<212> DNA
<213> Artificial Sequence

<220>
<223> Description of Artificial Sequence:DPK22-C/random

<400> 107
caccttggtc ccttggccga acgtathcgg cggcggacca cbctgctgac agtaatacac 60
tgc                                                                63

<210> 108
<211> 63
<212> DNA

<213> Artificial Sequence

<220>
<223> Description of Artificial Sequence:DPK22-C/random

<400> 108
caccttggtc ccttggccga acgtathcgg cggcggaccc ggctgctgac agtaatacac 60
tgc 63


<210> 109
<211> 63
<212> DNA
<213> Artificial Sequence

<220>
<223> Description of Artificial Sequence:DPK22-C/random

<400> 109
caccttggtc ccttggccga acgtacbcgg athathacca thctgctgac agtaatacac 60
tgc 63


<210> 110
<211> 63
<212> DNA
<213> Artificial Sequence

<220>
<223> Description of Artificial Sequence:DPK22-C/random

<400> 110
caccttggtc ccttggccga acgtacbcgg athathacca cbctgctgac agtaatacac 60
tgc 63


<210> 111
<211> 63
<212> DNA
<213> Artificial Sequence

<220>
<223> Description of Artificial Sequence:DPK22-C/random

<400> 111
caccttggtc ccttggccga acgtacbcgg athathaccc ggctgctgac agtaatacac 60

tgc                                                                63

<210> 112
<211> 63
<212> DNA
<213> Artificial Sequence

<220>
<223> Description of Artificial Sequence:DPK22-C/random

<400> 112
caccttggtc ccttggccga acgtacbcgg athacbacca thctgctgac agtaatacac 60
tgc                                                                63

<210> 113
<211> 63
<212> DNA
<213> Artificial Sequence

<220>
<223> Description of Artificial Sequence:DPK22-C/random

<400> 113
caccttggtc ccttggccga acgtacbcgg athacbacca cbctgctgac agtaatacac 60
tgc                                                                63

<210> 114
<211> 63
<212> DNA
<213> Artificial Sequence

<220>
<223> Description of Artificial Sequence:DPK22-C/random

<400> 114
caccttggtc ccttggccga acgtacbcgg athacbaccc ggctgctgac agtaatacac 60
tgc                                                                63

<210> 115
<211> 63
<212> DNA
<213> Artificial Sequence

<220>
<223> Description of Artificial Sequence:DPK22-C/random

<400> 115
caccttggtc ccttggccga acgtacbcgg athcggacca thctgctgac agtaatacac 60
tgc 63

<210> 116
<211> 63
<212> DNA
<213> Artificial Sequence

<220>
<223> Description of Artificial Sequence:DPK22-C/random

<400> 116
caccttggtc ccttggccga acgtacbcgg athcggacca cbctgctgac agtaatacac 60
tgc 63

<210> 117
<211> 63
<212> DNA
<213> Artificial Sequence

<220>
<223> Description of Artificial Sequence:DPK22-C/random

<400> 117
caccttggtc ccttggccga acgtacbcgg athcggaccc ggctgctgac agtaatacac 60
tgc 63

<210> 118
<211> 63
<212> DNA
<213> Artificial Sequence

<220>
<223> Description of Artificial Sequence:DPK22-C/random

<400> 118
caccttggtc ccttggccga acgtacbcgg acbathacca thctgctgac agtaatacac 60
tgc 63

<210> 119
<211> 63
<212> DNA
<213> Artificial Sequence

<220>
<223> Description of Artificial Sequence:DPK22-C/random

<400> 119
caccttggtc ccttggccga acgtacbcgg acbathacca cbctgctgac agtaatacac 60
tgc 63

<210> 120
<211> 63
<212> DNA
<213> Artificial Sequence

<220>
<223> Description of Artificial Sequence:DPK22-C/random

<400> 120
caccttggtc ccttggccga acgtacbcgg acbathaccc ggctgctgac agtaatacac 60
tgc 63

<210> 121
<211> 63
<212> DNA
<213> Artificial Sequence

<220>
<223> Description of Artificial Sequence:DPK22-C/random

<400> 121
caccttggtc ccttggccga acgtacbcgg acbacbacca thctgctgac agtaatacac 60
tgc 63

<210> 122
<211> 63
<212> DNA
<213> Artificial Sequence

<220>
<223> Description of Artificial Sequence:DPK22-C/random

<400> 122
caccttggtc ccttggccga acgtacbcgg acbacbacca cbctgctgac agtaatacac 60
tgc 63

<210> 123
<211> 63
<212> DNA
<213> Artificial Sequence

<220>
<223> Description of Artificial Sequence:DPK22-C/random

<400> 123
caccttggtc ccttggccga acgtacbcgg acbacbaccc ggctgctgac agtaatacac 60
tgc 63

<210> 124
<211> 63
<212> DNA
<213> Artificial Sequence

<220>
<223> Description of Artificial Sequence:DPK22-C/random

<400> 124
caccttggtc ccttggccga acgtacbcgg acbcggacca thctgctgac agtaatacac 60
tgc 63

<210> 125
<211> 63
<212> DNA
<213> Artificial Sequence

<220>
<223> Description of Artificial Sequence:DPK22-C/random

<400> 125
caccttggtc ccttggccga acgtacbcgg acbcggacca cbctgctgac agtaatacac 60
tgc 63

<210> 126
<211> 63
<212> DNA
<213> Artificial Sequence

<220>
<223> Description of Artificial Sequence:DPK22-C/random

<400> 126
caccttggtc ccttggccga acgtacbcgg acbcggaccc ggctgctgac agtaatacac 60
tgc 63

<210> 127
<211> 63
<212> DNA
<213> Artificial Sequence

<220>
<223> Description of Artificial Sequence:DPK22-C/random

<400> 127
caccttggtc ccttggccga acgtacbcgg cggathacca thctgctgac agtaatacac 60
tgc 63

<210> 128
<211> 63
<212> DNA
<213> Artificial Sequence

<220>
<223> Description of Artificial Sequence:DPK22-C/random

<400> 128
caccttggtc ccttggccga acgtacbcgg cggathacca cbctgctgac agtaatacac 60
tgc 63

<210> 129
<211> 63
<212> DNA
<213> Artificial Sequence

<220>

<223> Description of Artificial Sequence:DPK22-C/random

<400> 129
caccttggtc ccttggccga acgtacbcgg cggathaccc ggctgctgac agtaatacac 60
tgc 63

<210> 130
<211> 63
<212> DNA
<213> Artificial Sequence

<220>
<223> Description of Artificial Sequence:DPK22-C/random

<400> 130
caccttggtc ccttggccga acgtacbcgg cggacbacca thctgctgac agtaatacac 60
tgc 63

<210> 131
<211> 63
<212> DNA
<213> Artificial Sequence

<220>
<223> Description of Artificial Sequence:DPK22-C/random

<400> 131
caccttggtc ccttggccga acgtacbcgg cggacbacca cbctgctgac agtaatacac 60
tgc 63

<210> 132
<211> 63
<212> DNA
<213> Artificial Sequence

<220>
<223> Description of Artificial Sequence:DPK22-C/random

<400> 132
caccttggtc ccttggccga acgtacbcgg cggacbaccc ggctgctgac agtaatacac 60
tgc 63

<210> 133
<211> 63
<212> DNA
<213> Artificial Sequence

<220>
<223> Description of Artificial Sequence:DPK22-C/random

<400> 133
caccttggtc ccttggccga acgtacbcgg cggcggacca thctgctgac agtaatacac 60
tgc 63


<210> 134
<211> 63
<212> DNA
<213> Artificial Sequence

<220>
<223> Description of Artificial Sequence:DPK22-C/random

<400> 134
caccttggtc ccttggccga acgtacbcgg cggcggacca cbctgctgac agtaatacac 60
tgc 63


<210> 135
<211> 63
<212> DNA
<213> Artificial Sequence

<220>
<223> Description of Artificial Sequence:DPK22-C/random

<400> 135
caccttggtc ccttggccga acgtacbcgg cggcggaccc ggctgctgac agtaatacac 60
tgc 63


<210> 136
<211> 63
<212> DNA
<213> Artificial Sequence

<220>
<223> Description of Artificial Sequence:DPK22-C/random

<400> 136
caccttggtc ccttggccga acgtcggcgg athathacca thctgctgac agtaatacac 60
tgc 63

<210> 137
<211> 63
<212> DNA
<213> Artificial Sequence

<220>
<223> Description of Artificial Sequence:DPK22-C/random

<400> 137
caccttggtc ccttggccga acgtcggcgg athathacca cbctgctgac agtaatacac 60
tgc 63

<210> 138
<211> 63
<212> DNA
<213> Artificial Sequence

<220>
<223> Description of Artificial Sequence:DPK22-C/random

<400> 138
caccttggtc ccttggccga acgtcggcgg athathaccc ggctgctgac agtaatacac 60
tgc 63

<210> 139
<211> 63
<212> DNA
<213> Artificial Sequence

<220>
<223> Description of Artificial Sequence:DPK22-C/random

<400> 139
caccttggtc ccttggccga acgtcggcgg athacbacca thctgctgac agtaatacac 60
tgc 63

<210> 140

<211> 63
<212> DNA
<213> Artificial Sequence

<220>
<223> Description of Artificial Sequence:DPK22-C/random

<400> 140
caccttggtc ccttggccga acgtcggcgg athacbacca cbctgctgac agtaatacac 60
tgc                                                                63


<210> 141
<211> 63
<212> DNA
<213> Artificial Sequence

<220>
<223> Description of Artificial Sequence:DPK22-C/random

<400> 141
caccttggtc ccttggccga acgtcggcgg athacbaccc ggctgctgac agtaatacac 60
tgc                                                                63


<210> 142
<211> 63
<212> DNA
<213> Artificial Sequence

<220>
<223> Description of Artificial Sequence:DPK22-C/random

<400> 142
caccttggtc ccttggccga acgtcggcgg athcggacca thctgctgac agtaatacac 60
tgc                                                                63


<210> 143
<211> 63
<212> DNA
<213> Artificial Sequence

<220>
<223> Description of Artificial Sequence:DPK22-C/random

<400> 143
caccttggtc ccttggccga acgtcggcgg athcggacca cbctgctgac agtaatacac 60
tgc 63


<210> 144
<211> 63
<212> DNA
<213> Artificial Sequence

<220>
<223> Description of Artificial Sequence:DPK22-C/random

<400> 144
caccttggtc ccttggccga acgtcggcgg athcggaccc ggctgctgac agtaatacac 60
tgc 63


<210> 145
<211> 63
<212> DNA
<213> Artificial Sequence

<220>
<223> Description of Artificial Sequence:DPK22-C/random

<400> 145
caccttggtc ccttggccga acgtcggcgg acbathacca thctgctgac agtaatacac 60
tgc 63


<210> 146
<211> 63
<212> DNA
<213> Artificial Sequence

<220>
<223> Description of Artificial Sequence:DPK22-C/random

<400> 146
caccttggtc ccttggccga acgtcggcgg acbathacca cbctgctgac agtaatacac 60
tgc 63


<210> 147
<211> 63

<212> DNA
<213> Artificial Sequence

<220>
<223> Description of Artificial Sequence:DPK22-C/random

<400> 147
caccttggtc ccttggccga acgtcggcgg acbathaccc ggctgctgac agtaatacac 60
tgc 63


<210> 148
<211> 63
<212> DNA
<213> Artificial Sequence

<220>
<223> Description of Artificial Sequence:DPK22-C/random

<400> 148
caccttggtc ccttggccga acgtcggcgg acbacbacca thctgctgac agtaatacac 60
tgc 63


<210> 149
<211> 63
<212> DNA
<213> Artificial Sequence

<220>
<223> Description of Artificial Sequence:DPK22-C/random

<400> 149
caccttggtc ccttggccga acgtcggcgg acbacbacca cbctgctgac agtaatacac 60
tgc 63


<210> 150
<211> 63
<212> DNA
<213> Artificial Sequence

<220>
<223> Description of Artificial Sequence:DPK22-C/random

<400> 150
   caccttggtc ccttggccga acgtcggcgg acbacbaccc ggctgctgac agtaatacac 60
   tgc 63

<210> 151
<211> 63
<212> DNA
<213> Artificial Sequence

<220>
<223> Description of Artificial Sequence:DPK22-C/random

<400> 151
caccttggtc ccttggccga acgtcggcgg acbcggacca thctgctgac agtaatacac 60
tgc 63

<210> 152
<211> 63
<212> DNA
<213> Artificial Sequence

<220>
<223> Description of Artificial Sequence:DPK22-C/random

<400> 152
caccttggtc ccttggccga acgtcggcgg acbcggacca cbctgctgac agtaatacac 60
tgc 63

<210> 153
<211> 63
<212> DNA
<213> Artificial Sequence

<220>
<223> Description of Artificial Sequence:DPK22-C/random

<400> 153
caccttggtc ccttggccga acgtcggcgg acbcggaccc ggctgctgac agtaatacac 60
tgc 63

<210> 154
<211> 63
<212> DNA

<213> Artificial Sequence

<220>
<223> Description of Artificial Sequence:DPK22-C/random

<400> 154
caccttggtc ccttggccga acgtcggcgg cggathacca thctgctgac agtaatacac 60
tgc 63


<210> 155
<211> 63
<212> DNA
<213> Artificial Sequence

<220>
<223> Description of Artificial Sequence:DPK22-C/random

<400> 155
caccttggtc ccttggccga acgtcggcgg cggathacca cbctgctgac agtaatacac 60
tgc 63


<210> 156
<211> 63
<212> DNA
<213> Artificial Sequence

<220>
<223> Description of Artificial Sequence:DPK22-C/random

<400> 156
caccttggtc ccttggccga acgtcggcgg cggathaccc ggctgctgac agtaatacac 60
tgc 63


<210> 157
<211> 63
<212> DNA
<213> Artificial Sequence

<220>
<223> Description of Artificial Sequence:DPK22-C/random

<400> 157
caccttggtc ccttggccga acgtcggcgg cggacbacca thctgctgac agtaatacac 60

tgc                                                                63

<210> 158
<211> 63
<212> DNA
<213> Artificial Sequence

<220>
<223> Description of Artificial Sequence:DPK22-C/random

<400> 158
caccttggtc ccttggccga acgtcggcgg cggacbacca cbctgctgac agtaatacac 60
tgc                                                                63

<210> 159
<211> 63
<212> DNA
<213> Artificial Sequence

<220>
<223> Description of Artificial Sequence:DPK22-C/random

<400> 159
caccttggtc ccttggccga acgtcggcgg cggacbaccc ggctgctgac agtaatacac 60
tgc                                                                63

<210> 160
<211> 63
<212> DNA
<213> Artificial Sequence

<220>
<223> Description of Artificial Sequence:DPK22-C/random

<400> 160
caccttggtc ccttggccga acgtcggcgg cggcggacca thctgctgac agtaatacac 60
tgc                                                                63

<210> 161
<211> 63
<212> DNA
<213> Artificial Sequence

<220>
<223> Description of Artificial Sequence:DPK22-C/random

<400> 161
caccttggtc ccttggccga acgtcggcgg cggcggacca cbctgctgac agtaatacac 60
tgc                                                               63


<210> 162
<211> 63
<212> DNA
<213> Artificial Sequence

<220>
<223> Description of Artificial Sequence:DPK22-C/random

<400> 162
caccttggtc ccttggccga acgtcggcgg cggcggaccc ggctgctgac agtaatacac 60
tgc                                                               63


<210> 163
<211> 742
<212> DNA
<213> Artificial Sequence

<220>
<223> Description of Artificial Sequence:DP47-DPK22
      Sequence

<400> 163
ccatggccga ggtgcagctg ttggagtctg ggggaggttt ggtacagcct ggggggtccc 60
tgagactctc ctgtgcagcc tctggattca cctttagtag ttatgccatg agctgggtcc 120
gccaagctcc agggaagggg ctggagtggg tctctgctat tagtgggagt ggtggtagca 180
catattatgc agactctgtg aagggccgat tcaccatctc cagagacaac tccaagaaca 240
ccctgtatct gcaaatgaac agtctgagag ccgaggacac ggccgtatat tactgtgcga 300
aannnnnnnn nnnntttgac tactggggcc agggaaccct ggtcaccgtc tccagtggct 360
cgagtggtgg aggcggttca ggcggaggtg gctctggcgg tagtgcacag gaaattgtgt 420
tgacgcagtc tccaggcacc ctgtctttgt ctccagggga aagagccacc ctctcctgca 480
gggccagtca gagtgttagc agcagctact tagcctggta ccagcagaaa cctggccagg 540
ctcccaggct cctcatctat ggtgcatcca gcagggccac tggcatccca gacaggttca 600
gtggcagtgg gtctgggaca gacttcactc tcaccatcag cagactggag cctgaagatt 660
ttgcagtgta ttactgtcag cagnnnggtn nnnnnccgnn nacgttcggc caagggacca 720
aggtggaaat caaagcggcc gc                                          742

<210> 164
<211> 364
<212> DNA
<213> Homo sapiens

<400> 164
ccatggccga ggtgcagctg ttggagtctg ggggaggctt ggtacagcct ggggggtccc 60
tgagactctc ctgtgcagcc tctggattca cctttagcag ctatgccatg agctgggtcc 120
gccaggctcc agggaagggg ctggagtggg tctcagctat tagtggtagt ggtggtagca 180
catactacgc agactccgtg aagggccggt tcaccatctc cagagacaat tccaagaaca 240
cgctgtatct gcaaatgaac agcctgagag ccgaggacac ggccgtatat tactgtgcga 300
aannnnnnnn nnnntttgac tactggggcc agggaaccct ggtcaccgtc tcgagtggct 360
cgag 364

<210> 165
<211> 36
<212> DNA
<213> Artificial Sequence

<220>
<223> Description of Artificial Sequence:DP47-N/Nco

<400> 165
catgccatgg ccgaggtgca gctgttggag tctggg 36

<210> 166
<211> 23
<212> DNA
<213> Artificial Sequence

<220>
<223> Description of Artificial Sequence:DP47-C

<400> 166
tttcgcacag taatatacgg ccg 23

<210> 167
<211> 340
<212> DNA
<213> Homo sapiens

<400> 167

gtgcacagga aattgtgttg acgcagtctc caggcaccct gtctttgtct ccaggggaaa 60
gagccaccct ctcctgcagg gccagtcaga gtgttagcag cagctactta gcctggtacc 120
agcagaaacc tggccaggct cccaggctcc tcatctatgg tgcatccagc agggccactg 180
gcatcccaga caggttcagt ggcagtgggt ctgggacaga cttcactctc accatcagca 240
gactggagcc tgaagatttt gcagtgtatt actgtcagca gnnnggtnnn nnnccgnnna 300
cgttcggcca agggaccaag gtggaaatca aagcggccgc 340

<210> 168
<211> 35
<212> DNA
<213> Artificial Sequence

<220>
<223> Description of Artificial Sequence:DPK22-N/Apa

<400> 168
ggtagtgcac aggaaattgt gttgacgcag tctcc 35

<210> 169
<211> 21
<212> DNA
<213> Artificial Sequence

<220>
<223> Description of Artificial Sequence:DPK22-C

<400> 169
acagtaatac actgcaaaat c 21

<210> 170
<211> 302
<212> DNA
<213> Homo sapiens

<400> 170
   ccatggccga ggtgcagctg ttggagtctg ggggaggtgt ggtacggcct ggggggtccc 60
   tgagactctc ctgtgcagcc tctggattca cctttgatga ttatggcatg agctgggtcc 120
   gccaagctcc agggaagggg ctggagtggg tctctggtat taattggaat ggtggtagca 180
   caggttatgc agactctgtg aagggccgat tcaccatctc cagagacaac gccaagaact 240
   ccctgtatct gcaaatgaac agtctgagag ccgaggacac ggccgtatat tactgtgcga 300
   aa 302

<210> 171
<211> 34
<212> DNA
<213> Artificial Sequence

<220>
<223> Description of Artificial Sequence:DP47-5'-1

<400> 171
gtctggggga ggtttggtac agcctggggg gtcc                                34

<210> 172
<211> 34
<212> DNA
<213> Artificial Sequence

<220>
<223> Description of Artificial Sequence:DP47-3'-1

<400> 172
cccagctcat ggcataacta ctaaaggtga atcc                                34

<210> 173
<211> 47
<212> DNA
<213> Artificial Sequence

<220>
<223> Description of Artificial Sequence:DP47-5'-2

<400> 173
gggtctctgc tattagtggg agtggtggta gcacatatta tgcagac                  47

<210> 174
<211> 35
<212> DNA
<213> Artificial Sequence

<220>
<223> Description of Artificial Sequence:DP47-3'-2

<400> 174
gcagatacag ggtgttcttg gagttgtctc tggag                               35

<210> 175
<211> 302
<212> DNA
<213> Artificial Sequence

<220>
<223> Description of Artificial Sequence:Mutated DP47

<400> 175
ccatggccga ggtgcagctg ttggagtctg ggggaggttt ggtacagcct ggggggtccc 60
tgagactctc ctgtgcagcc tctggattca cctttagtag ttatgccatg agctgggtcc 120
gccaagctcc agggaagggg ctggagtggg tctctgctat tagtgggagt ggtggtagca 180
catattatgc agactctgtg aagggccgat tcaccatctc cagagacaac tccaagaaca 240
ccctgtatct gcaaatgaac agtctgagag ccgaggacac ggccgtatat tactgtgcga 300
aa 302

<210> 176
<211> 51
<212> DNA
<213> Artificial Sequence

<220>
<223> Description of Artificial Sequence:DP47-C/Xho

<400> 176
accactcgag ccactggaga cggtgaccag ggttccctgg ccccagtagt c 51

<210> 177
<211> 44
<212> DNA
<213> Artificial Sequence

<220>
<223> Description of Artificial Sequence:DPK22-C/Not

<400> 177
acttgcggcc gctttgattt ccaccttggt cccttggccg aacg 44